# EUROPEAN PATENT APPLICATION

(11) **EP 4 431 527 A1**
(43) Date of publication of application: **18.09.2024**
(21) Application number: 23162746.4
(22) Date of filing: 17.03.2023
(51) Int. Cl.: C07K 16/28, C07K 14/16

(54) **FUSION PROTEIN FOR LOCALIZED ACTIVATION OF T-CELLS**

(71) Applicant: Medizinische Hochschule Hannover, 30625 Hannover (DE)
(72) Inventor: Kühnel, Florian, 30163 Hannover (DE); Wirth, Thomas, 30163 Hannover (DE); Peter, Malin, 30163 Hannover (DE)
(74) Representative: Taruttis, Stefan Georg

(57) **Abstract**

The invention provides a fusion protein and a nucleic acid construct encoding the fusion protein, preferably an oncolytic viral particle that contains the nucleic acid construct encoding the fusion protein, wherein the fusion protein from N-terminus to C-terminus comprises or consists of a secretory leader peptide - anti-CD3-antibody section - hinge - HIV-Tat.

## Description

The present invention relates to a fusion protein for localized activation of cytotoxic T-cells, especially for use in the treatment of solid tumours, and to viral particles that contain a nucleic acid construct that encodes the fusion protein, which viral particles are for use in the treatment of solid tumours. Presence of the fusion protein in a tumour has the advantage of activating an immune response of cytotoxic T-cells against the tumour, especially T-cells specifically directed against a tumour antigen. The fusion protein of the invention, e.g. when expressed from a nucleic acid construct contained in a viral particle, has the advantage of activating T-cells at the location of expression of the fusion protein, especially when expressed within tumour tissue, with little or no activity to activate T-cells outside the tumour in which the fusion protein is expressed. The fusion protein of the invention has furthermore the advantage to bind to tissue, e.g. tumour tissue, and to activate T-cells at the site of its presence, independent of the presence of a particular tumour-specific antigen in the tissue. Accordingly, the fusion protein can be used in the treatment of tumours in the absence of a determination of tumour-specific antigens, and for use in the treatment of tumours that are devoid of a tumour-specific antigen.

Preferably, the fusion protein of the invention is for use in activating T-cells within a tissue, e.g. for use in the treatment of a tumour, wherein the fusion protein is present, e.g. expressed in or introduced into the tissue, e.g. tumour tissue, for activating T-cells in this tissue.

The fusion protein preferably is encoded by a nucleic acid construct contained in an oncolytic viral particle, such that administration of the viral particle to a human results in pre-dominant presence of the viral particle in the vicinity of or within tumour tissue and expression of thefusion protein in the vicinity of or within tumour tissue. Generally preferred, the fusion protein is encoded by a nucleic acid construct packaged in a viral particle for transient expression only. The viral particle preferably is an oncolytic virus.

### State of the art

Liao et al., Cancer Gene Therapy (2003) 10, 779-790, for use in tumour treatment describe expression of fusion proteins containing an anti-CD3-scFv, a linker and a transmembrane domain in cells for activating T-cells against the cells expressing the fusion protein. The fusion protein was produced in cultivated cells by transient transfection using lipofectamine in vitro. For activating T-cells, this publication concludes that it is important to reduce shedding of such fusion protein from cells that express this fusion protein.

Liao et Roffler, Gene Therapy (2000) 7, 339-347, describe activation of cytotoxic T-cells by expressing a fusion protein of anti-CD3-scFv - hinge region of IgG - transmembrane domain and cytosolic domain of CD80 as a surface-bound protein after transfection into in vitro cultivated tumour cells.

WO2021/239586 A1 describes oncolytic viruses which have an inactivated E4 orf3 and a mutated E4 orf4.

### Object of the invention

It is an object of the invention to provide an alternative, preferably an improved fusion protein for use in the treatment of tumours, especially for activating cytotoxic T-cells, and a carrier for the fusion protein for use in the treatment of tumours.

### Description of the invention

The invention achieves the object by the features of the claims and especially by a fusion protein and a nucleic acid construct encoding the fusion protein, preferably an oncolytic viral particle that contains the nucleic acid construct encoding the fusion protein, wherein the fusion protein from N-terminus to C-terminus comprises or consists of a secretory leader peptide - anti-CD3-antibody section - hinge - HIV-Tat, with optionally a Tag-domain between the scFv and the hinge, wherein the anti-CD3-antibody section preferably is an anti-CD3-scFv domain, e.g. a single-chain antibody of a variable light chain (VL), a linker and a variable heavy chain (VH) VL-linker-VH or VH-linker-VL, which linker is e.g. a Gly-Ser-linker, wherein the Tag-domain is e.g. a mycHis-Tag that can be detected by an anti-Myc-antibody and that contains a His-oligomer for affinity purification, wherein the HIV-Tat is an electropositive section of the HIV-Tat protein from the HIV. Generally herein, the anti-CD3-antibody section is represented by an anti-CD3 scFv domain.

It was found that the anti-CD3-scFv domain of the fusion protein results in activation of cytotoxic T-cells inside a solid tumour and/or results in invasion of active cytotoxic T-cells into a solid tumour, wherein the activation is not limited to those tumour cells that express the fusion protein, but the activation of cytotoxic T-cells also occurs in the tumour-tissue vicinity of those tumour cells that express the fusion protein. It is believed that the secretion of the fusion protein, as caused by its secretory leader peptide and lack of a transmembrane domain and lack of intracellular domains also results in spreading of the fusion protein within tumour tissue, whereas the HIV-Tat domain prevents an unlimited distribution of the fusion protein but results in a gradual diffusion of the fusion protein with preference into tissue within the direct vicinity of cells expressing the fusion protein. It is currently assumed that the HIV-Tat domain after secretion from expressing cells results in the fusion protein sticking to tissue, e.g. tumour tissue, and presence of the fusion protein activates cytotoxic T-cells. Therefore, the fusion protein has the advantage of activating cytotoxic T-cells also in tumour cells that are not transduced by viral particles to express the fusion protein, but which tumour cells are only in the vicinity of cells expressing the fusion protein. Because the cell-binding properties of the fusion protein containing the HIV-Tat domain are not dependent on presence of a specific antigen in the tissue, i.e. the cell-binding properties of the fusion protein are not restricted to a specific tumor antigen, expression of the fusion protein does not limit the broad range applicability of the carrier viral particle encoding the fusion protein. Accordingly, a viral particle, preferably an oncolytic virus, encoding the fusion protein is suitable for use in the activation of T-cells, e.g. for use in the treatment of a tumour, in the tissue present in the vicinity of the cells that are infected by the viral particle. Therein, the localization of the activation of T-cells is determined by the specificity of the viral particle, e.g. specificity for a target tissue, e.g. tumour tissue.

As the fusion protein is secreted from cells that are infected by a virus encoding the fusion protein, the activity of the fusion protein to activate T-cells, especially within tissue in the vicinity of the infected cells, is independent from the type of the virus.

A viral particle, preferably an oncolytic viral particle, that contains a nucleic acid construct encoding the fusion protein is used as a carrier, resulting in expression of the fusion protein by cells, preferably tumour cells, of the recipient human. As an alternative to a viral particle, liposomes can be used as a carrier, the liposomes containing a nucleic acid encoding the fusion protein. As an embodiment, a nucleic acid construct encoding the fusion protein is contained in liposomes, wherein the liposomes preferably contain a protein providing for tumour-specificity, e.g. the liposomes may have a liposome-membrane bound tumour-specific ligand, e.g. an antibody that is specific for a tumour-specific antigen, which antibody is bound to the liposomal membrane. Alternatively or additionally, the liposomes can be for use in direct intra-tumoural injection.

Generally herein, protein sequences and arrangements of domains of the fusion protein are described from N-terminus to C-terminus.

An exemplary fusion protein consisting of secretory leader peptide - anti-CD3-scFv - Tag - hinge - HIV-Tat is 
MALLLCFVLLCGVVDFARSLSASDIQLTQSPAIMSASPGEKVTMTCRASSSVSYMNW
YQQKSGTSPKRWIYDTSKVASGVPYRFSGSGSGTSYSLTISSMEAEDAATYYCQQWS
SNPLTFGAGTKLELKGGGGSGGGGSGGGGSDIKLQQSGAELARPGASVKMSCKTSG
YTFTRYTMHWVKQRPGQGLEWIGYINPSRGYTNYNQKFKDKATLTTDKSSSTAYMQ
LSSLTSEDSAVYYCARYYDDHYCLDYWGQGTTLTVSSEQKLISEEDLNMHTGHHHH
HHPRGPTIKPCPPCKECHKCPAPNLEGGPSVFIFPPNIKDVLMISLTPKVTCVVVDVSE
DDPDVRISWFVNNVEVHTAQTQTHREDYNSTIRVVSALPIQHQDWMSGKEFKCKVN
NKDLPSPIERTISKIKGLVRAPQVYILPPPAEQLSRKDVSLTCLVVGFNPGDISVEWTSN
GHTEENYKDTAPVLDSDGSYFIYSKLDIKTSKWEKTDSFSCNVRHEGLKNYYLKKTIS
RTPGKGRKKRRQRRR
 (SEQ ID NO: 1), containing an anti-CD3 scFV as VL-hinge-VH, specific for the human CD3, with a 6x His tag as an optional Tag, and 
MALLLCFVLLCGVVDFARSLSASDIKLQQSGAELARPGASVKMSCKTSGYTFTRYTM
HWVKQRPGQGLEWIGYINPSRGYTNYNQKFKDKATLTTDKSSSTAYMQLSSLTSEDS
AVYYCARYYDDHYCLDYWGQGTTLTVSSGGGGSGGGGSGGGGSDIQLTQSPAIMSA
SPGEKVTMTCRASSSVSYMNWYQQKSGTSPKRWIYDTSKVASGVPYRFSGSGSGTS
YSLTISSMEAEDAATYYCQQWSSNPLTFGAGTKLELKEQKLISEEDLNMHTGHHHHH
HPRGPTIKPCPPCKECHKCPAPNLEGGPSVFIFPPNIKDVLMISLTPKVTCVVVDVSED
DPDVRISWFVNNVEVHTAQTQTHREDYNSTIRVVSALPIQHQDWMSGKEFKCKVNN
KDLPSPIERTISKIKGLVRAPQVYILPPPAEQLSRKDVSLTCLVVGFNPGDISVEWTSNG
HTEENYKDTAPVLDSDGSYFIYSKLDIKTSKWEKTDSFSCNVRHEGLKNYYLKKTISR
TPGKGRKKRRQRRR
 (SEQ ID NO: 3), containing the anti-CD3 scFV as VH-hinge-VL, specific for the human CD3, with a 6x His tag as an optional Tag. The fusion protein of the invention can have an amino acid sequence having a sequence identity or homology of at least 80%, of at least 85%, of at least 90%, of at least 95% to one of, or to two of the sequences SEQ ID NO: 1, and SEQ ID NO: 3, optionally with an additional oligomerizing domain, preferably in combination with a hydrophilic spacer, arranged between the hinge and the HIV-Tat, e.g. an amino acid sequence having a sequence identity or homology of at least 80%, of at least 85%, of at least 90%, of at least 95% to one of, or to two of the sequences SEQ ID NO: 2, and SEQ ID NO: 4.

In an embodiment, the fusion protein can consist of a secretory leader peptide - anti-CD3-antibody section - hinge - HIV-Tat.

In a preferred embodiment, the fusion protein between its hinge and HIV-Tat contains an oligomerizing domain that results in e.g. dimerization, trimerization of secreted fusion proteins. A preferred oligomerizing domain is the fibritin domain. In this embodiment, the fusion protein from N-terminus to C-terminus comprises or consists of a secretory leader peptide - anti-CD3 antibody section - hinge - oligomerizing domain - HIV-Tat, with optionally a Tag-domain between the scFv and the hinge, wherein the anti-CD3 antibody section is an anti-CD3-scFv is a single-chain antibody of a variable light chain (VL), a linker and a variable heavy chain (VH) in the arrangement of VL-linker-VH or VH-linker-VL, which linker is e.g. a Gly-Ser-linker, wherein the Tag-domain is e.g. a MycHis-Tag that can be detected by an anti-Myc-antibody and that contains a His-oligomer for affinity purification by metal ion affinity, wherein the HIV-Tat is an electropositive section of the HIV-Tat domain from the HIV.

An exemplary fusion protein consisting of secretory leader peptide - anti-CD3-scFv - Tag - hinge - oligomerizing domain - HIV-Tat is 
MALLLCFVLLCGVVDFARSLSASDIQLTQSPAIMSASPGEKVTMTCRASSSVSYMNW
YQQKSGTSPKRWIYDTSKVASGVPYRFSGSGSGTSYSLTISSMEAEDAATYYCQQWS
SNPLTFGAGTKLELKGGGGSGGGGSGGGGSDIKLQQSGAELARPGASVKMSCKTSG
YTFTRYTMHWVKQRPGQGLEWIGYINPSRGYTNYNQKFKDKATLTTDKSSSTAYMQ
LSSLTSEDSAVYYCARYYDDHYCLDYWGQGTTLTVSSEQKLISEEDLNMHTGHHHH
HHPRGPTIKPCPPCKECHKCPAPNLEGGPSVFIFPPNIKDVLMISLTPKVTCVVVDVSE
DDPDVRISWFVNNVEVHTAQTQTHREDYNSTIRVVSALPIQHQDWMSGKEFKCKVN
NKDLPSPIERTISKIKGLVRAPQVYILPPPAEQLSRKDVSLTCLVVGFNPGDISVEWTSN
GHTEENYKDTAPVLDSDGSYFIYSKLDIKTSKWEKTDSFSCNVRHEGLKNYYLKKTIS
RTPGKGYIPEAPRDGQAYVRKDGEWVLLSTFLSPAGGGGSGGRKKRRQRRR
 (SEQ ID NO: 2), containing the anti-CD3 scFV as VH-hinge-VL, specific for the human CD3, with a 6x His tag as an optional Tag, with the fibritin domain as the oligomerizing domain, and containing a hydrophilic spacer section between the oligomerizing domain and HIV-Tat, and 
MALLLCFVLLCGVVDFARSLSASDIKLQQSGAELARPGASVKMSCKTSGYTFTRYTM
HWVKQRPGQGLEWIGYINPSRGYTNYNQKFKDKATLTTDKSSSTAYMQLSSLTSEDS
AVYYCARYYDDHYCLDYWGQGTTLTVSSGGGGSGGGGSGGGGSDIQLTQSPAIMSA
SPGEKVTMTCRASSSVSYMNWYQQKSGTSPKRWIYDTSKVASGVPYRFSGSGSGTS
YSLTISSMEAEDAATYYCQQWSSNPLTFGAGTKLELKEQKLISEEDLNMHTGHHHHH
HPRGPTIKPCPPCKECHKCPAPNLEGGPSVFIFPPNIKDVLMISLTPKVTCVVVDVSED
DPDVRISWFVNNVEVHTAQTQTHREDYNSTIRVVSALPIQHQDWMSGKEFKCKVNN
KDLPSPIERTISKIKGLVRAPQVYILPPPAEQLSRKDVSLTCLVVGFNPGDISVEWTSNG
HTEENYKDTAPVLDSDGSYFIYSKLDIKTSKWEKTDSFSCNVRHEGLKNYYLKKTISR
TPGKGYIPEAPRDGQAYVRKDGEWVLLSTFLSPAGGGGSGGRKKRRQRRR
 (SEQ ID NO: 4), containing the anti-CD3 scFV as VH-hinge-VL, specific for the human CD3, with a 6x His tag as an optional Tag, and with the fibritin domain as the oligomerizing domain, and containing a hydrophilic spacer section between the oligomerizing domain and HIV-Tat. Generally, arrangement of a hydrophilic spacer between the oligomerizing domain and HIV-Tat is optional, and preferred. The fibritin domain results in trimerization of the secreted fusion protein. It was found that the oligomerization of the fusion protein results in a higher efficacy of activation of T-cells compared to a fusion protein without oligomerization domain. As an alternative to the fibritin domain, GCN4, especially its human homolog, the activating transcription factor 4 (ATF4), can be contained in the fusion protein.

Generally, the fusion protein can be devoid of the Tag, as the Tag only serves as an epitope for an analytical antibody or, in the case of the 6x His tag, the Tag serves for enabling Ni-affinity purification of the fusion protein.

The hinge generally preferably has an amino acid sequence having sequence identity or homology of at least 80%, of at least 85%, of at least 90%, of at least 95% to at least one of amino acids 285..520 of SEQ ID NO: 1, amino acids 285..520 of SEQ ID NO: 2, amino acids 285..520 of SEQ ID NO: 3, and amino acids 285..520 of SEQ ID NO: 4.

The fibritin domain generally preferably has an amino acid sequence having sequence identity or homology of at least 80%, of at least 85%, of at least 90%, of at least 95% to at least one of amino acids 521..550 of SEQ ID NO: 2, and amino acids 521..550 of SEQ ID NO: 4.

The HIV-Tat domain generally preferably has an amino acid sequence having sequence identity or homology of at least 80%, of at least 85%, of at least 90%, of at least 95% to at least one of amino acids 521..530 of SEQ ID NO: 1, amino acids 557..566 of SEQ ID NO: 2, amino acids 521..530 of SEQ ID NO: 3, and amino acids 557..566 of SEQ ID NO: 4.

The anti-CD3 scFv generally preferably has an amino acid sequence having a sequence identity or homology of its variable light chain of at least 80%, of at least 85%, of at least 90%, of at least 95% to one of the variable light chain sections of one of SEQ ID NO: 1, SEQ ID NO: 2, SEQ ID NO: 3 and SEQ ID NO: 4, and a sequence identity or homology of its variable heavy chain of at least 80%, of at least 85%, of at least 90%, of at least 95% to one of the variable heavy chain sections of one of SEQ ID NO: 1, SEQ ID NO: 2, SEQ ID NO: 3 and SEQ ID NO: 4.

Optionally, the fusion protein is encoded on a nucleic acid construct under the translational control of an IRES (internal ribosome entry site), which is a combination of an IRES and the fusion protein encoding nucleic acid construct is arranged in 3' of a viral protein, e.g. E1B, such that transcription of the mRNA encoding the fusion protein is under control of a viral promoter, e.g. the E1B promoter, and translation of the fusion protein is dependent on the IRES.

Preferably, the nucleic acid construct encoding the fusion protein of the invention is arranged in expression units that avoid strong expression to overexpression of the fusion protein. A preferable weak expression of the fusion protein can be achieved by an expression unit that combines a viral (e.g. E1B) or a physiological promoter with an IRES motif for expression control of the fusion protein of the invention which leads to reduced expression levels of the gene located 3' of the IRES motif. In the combination of a promoter with an IRES that controls the translation of the fusion protein encoding nucleic acid, the promoter can be any promoter, e.g. a strong, a medium, or a weak promoter, e.g. a physiological or viral promoter. An exemplary strong promoter is the EF1 alpha promoter. Alternatively, the fusion protein of the invention can preferably be expressed under direct control of a weak promoter, which in a human cell, preferably in a human tumour cell, has a lower activity than the strong physiological phosphoglycerate kinase (PGK) promotor, such as a promoter having the strength of a glycerol aldehyde-3-phosphate dehydrogenase promoter, or a tumor-specific promoter. Accordingly, the nucleic acid section encoding the fusion protein preferably is under direct control of a weak promoter, such as the phosphoenol pyruvate carboxykinase promoter, a glycerol aldehyde-3-phosphate dehydrogenase promoter, or a tumour-specific promoter, each preferably of human origin.

The sequence listing represents the amino acid sequences from N-terminus to C-terminus and encoding nucleic acid sequences from 5' to 3' of fusion proteins of the invention, in detail in SEQ ID NO: 1 a fusion protein (anti-CD3h-TAT) having a secretory leader, an anti-CD3 scFv directed against human CD3 (anti-human CD3 scFv) in the arrangement of variable light chain-linker-variable heavy chain, a Myc-tag, a His-tag, a hinge domain, and HIV-Tat, SEQ ID NO: 2 a fusion protein (anti-CD3h-TAT-Trimer) having a secretory leader, an anti-CD3 scFv directed against human CD3 (anti-human CD3 scFv) in the arrangement of variable light chain-linker-variable heavy chain, a Myc-tag, a His-tag, a hinge domain, a fibritin domain, a hydrophilic spacer, and HIV-Tat, SEQ ID NO: 3 a fusion protein (anti-CD3h-TAT) having a secretory leader, an anti-CD3 scFv directed against human CD3 (anti-human CD3 scFv) in the arrangement of variable heavy chain-linker-variable light chain, a Myc-tag, a His-tag, a hinge domain, and HIV-Tat, SEQ ID NO: 4 a fusion protein (anti-CD3h-TAT-Trimer) having a secretory leader, an anti-CD3 scFv directed against human CD3 (anti-human CD3 scFv) in the arrangement of variable heavy chain-linker-variable light chain, a Myc-tag, a His-tag, a hinge domain, a fibritin domain, a hydrophilic spacer, and HIV-Tat, SEQ ID NO: 5 the coding DNA for a fusion protein of SEQ ID NO: 1 (anti-CD3h-TAT), SEQ ID NO: 6 the coding DNA for a fusion protein of SEQ ID NO: 2 (anti-CD3h-TAT-Trimer), SEQ ID NO: 7 the coding DNA for a fusion protein of SEQ ID NO: 3 (anti-CD3h-TAT), SEQ ID NO: 8 the coding DNA for a fusion protein of SEQ ID NO: 4 (anti-CD3h-TAT-Trimer), SEQ ID NO: 9 a fusion protein (anti-CD3m-TAT) having a secretory leader, an anti-CD3 scFv directed against murine CD3 (anti-murine CD3 scFv) in the arrangement of variable light chain-linker-variable heavy chain, a Myc-tag, a His-tag, a hinge domain, and HIV-Tat, SEQ ID NO: 10 a fusion protein (anti-CD3m-TAT-Trimer) having a secretory leader, an anti-CD3 scFv directed against murine CD3 (anti-murine CD3 scFv) in the arrangement of variable light chain-linker-variable heavy chain, a Myc-tag, a His-tag, a hinge domain, a fibritin domain, a hydrophilic spacer, and HIV-Tat, SEQ ID NO: 11 the coding DNA for a fusion protein of SEQ ID NO: 9 (anti-CD3m-TAT), SEQ ID NO: 12 the coding DNA for a fusion protein of SEQ ID NO: 10 (anti-CD3m-TAT), SEQ ID NO: 13 the coding DNA for a preferred viral vector which encodes a fusion protein of the invention at nucleotides No. 4339..5919, which coding sequence for the fusion protein can be replaced by a coding sequence for any fusion protein of the invention, SEQ ID NO: 71 the coding DNA for a preferred viral vector which encodes a fusion protein of the invention at nucleotides No. 4339..6027 in the embodiment containing a fibritin domain as a trimerization domain, which coding sequence for the fusion protein can be replaced by a coding sequence for any fusion protein of the invention.

An oncolytic viral particle is e.g. a herpes simplex virus, a reovirus, Newcastle disease virus, vesicular stomatitis virus, parvovirus H1, measles virus, vaccinia virus, maraba virus, polio virus, coxsackievirus, in each case attenuated and/or inactivated, preferably an adenovirus, especially an adenovirus serotype 5, with a deleted or inactivated gene E1B55k and/or deleted or inactivated gene E1B 19k, the inactivation being introduced e.g. by mutation, an adenovirus with a deletion in the Rb binding domain of E1A, also referred to as delta24 adenovirus or delta 922-947 adenovirus, adenovirus containing a tumour specific promoter controlling expression of E1A, which is e.g. the telomerase promoter, the AFP promoter, PSA promoter, DF1/Muc promoter, tyrosinase promoter, a HIF1alpha-dependent promoter, or a E2F/C-myc-dependent promoter.

A preferred oncolytic virus is a viral particle according to WO2021/239586 A1, the contents of which are incorporated herein by reference. A preferred embodiment of an oncolytic viral particle encoding a fusion protein of the invention is an adenovirus, which preferably contains the nucleic acid sequence of SEQ ID NO: 13, wherein the sequence encoding the fusion protein of nucleotides No. 4405-5985, which contains an anti-murine CD3 scFv, can optionally be replaced by a sequence encoding the fusion protein having an anti-human CD3 scFv, e.g. one of the embodiments of the fusion protein containing the anti-human CD3-scFv - linker - HIV-Tat, optionally with an oligomerization domain and/or with a hydrophilic spacer between the linker and the HIV-Tat domain. Preferably, the adenovirus encoding DNA from 5' to 3' comprises or consists of a left-end inverted terminal repeat (L-ITR), a first promoter, a coding sequence for E1A, an E1B promoter controlling a coding sequence for E1B, an expression cassette for at least one effector molecule, preferably an expression cassette encoding inhibitory RNA (RNAi) that are specific for RNAi target sites within the E1A encoding sequence and/or within the E1B promoter and/or within the E1B encoding sequence, vector backbone sequence, and on the opposite DNA strand an expression cassette for E4 comprising the orf4, the expression cassette for E4 containing in functional relationship a promoter driving expression of E4, and a right-end inverted terminal repeat (R-ITR). The arrangement of the L-ITR, E1A, a promoter controlling expression of E1B, preferably of an expression cassette for at least one effector molecule on one DNA strand, and preferably of coding sequences for inhibitory RNA under the control of a promoter which is activated by the presence of p53, e.g. the prMinRGC promoter, on one DNA strand, and arrangement of the expression cassette for E4 on the opposite DNA strand corresponds to the arrangement of the R-ITR, E4-promoter, and of the orfs constituting the coding sequence for E4 from 5' to 3' on the opposite strand. The expression cassette for an E4-orf4 encoded protein is arranged on the DNA strand opposite to the strand encoding the L-ITR, the first promoter, the E1A encoding sequence and the E1B promoter and the E1B encoding sequence. The L-ITR is arranged at one terminus of the DNA and the R-ITR is arranged at the terminus of the DNA opposite of the L-ITR.

The first promoter preferably is CMVs (nucleotides No. 380-625 of SEQ ID NO: 13), which has the advantage of becoming activated following infection of a cell with a time delay that allows for the effective inhibition of the E1A encoding sequence, the E1B promoter and of the E1B encoding sequence by inhibitory RNA molecules that are expressed under the control of prMinRGC.

Preferably, the coding sequence for E1A, the E1B promoter, and the coding sequence for E1B contain RNAi target sites for binding inhibitory RNA, e.g. shRNA, and the adenovirus encoding DNA contains an expression cassette for inhibitory RNA, e.g. for shRNA, which bind to the RNAi target sites, which expression cassette preferably is under the control of a promoter which is activated by presence of p53, e.g. the promoter prMinRGC. The coding sequences for inhibitory RNA, which inhibit expression of the adenoviral genes, e.g. of E1A, E1B, E4, pTP, Pol, are preferably expressed under the control of the prMinRGC promoter. The prMinRGC promoter is preferred as the promoter which is activated by the presence of p53.

Preferably, the viral particle is encoded by and contains a nucleic acid sequence of SEQ ID NO: 13 or of SEQ ID NO: 71.

As an alternative to a viral particle, a nucleic acid construct encoding the fusion protein can be contained in a liposome used as a carrier or the nucleic acid construct can be associated with a synthetic carrier particle. Generally preferred, a carrier containing a nucleic acid construct encoding the fusion protein is provided in a formulation suitable for direct injection into tumour tissue, and such a carrier is preferably used for direct injection into tumour tissue.

Generally, the fusion protein is a soluble protein, i.e. it is devoid of a transmembrane domain, and the fusion protein is secreted from the cell that contains a nucleic acid construct encoding the fusion protein.

The invention is now described by examples with reference to the figures, which show in
- Fig. 1A - 1D flow cytometry (FACS) results for the activation markers CD25 and CD69 on murine CD4+ T-cells and on CD8+ T-cells for CD90.2+ leukocytes in presence of fusion protein of the invention,
- Fig. 2 killing rates of murine cancer cells by T-cells in presence of fusion protein of the invention,
- Fig. 3A - 3D data for binding of human CD4+ T-cells by the fusion protein of the invention,
- Fig. 4A - 4E data for binding of human CD8+ T-cells by the fusion protein of the invention,
- Fig. 5A - 5B data for activation of both human CD4+ and CD8+ T-cells for anti-CD69 antibody staining by embodiments of the fusion protein,
- Fig. 6A - 6B by intracellular cytokine staining for TNFα cytokine production by human CD4+ and CD8+ T-cells incited by the fusion protein,
- Fig. 7A a schematic of a nucleic acid sequence encoding preferred a viral particle with a fusion protein of the invention,
- Fig. 7B data for increased tumour regression in mice caused by expression of the fusion protein of the invention,
- Fig. 7C data for improved survival of tumour bearing mice caused by expression of the fusion protein of the invention,
- Fig. 8A a schematic of nucleic acid constructs for expression of the fusion protein of the invention,
- Fig. 8B Western blots of culture supernatants of 293 T-cells expressing fusion proteins of the invention under the control of a weak or of a strong promoter,
- Fig. 8C data for improved survival of tumour bearing mice caused by expression of the fusion protein of the invention,
- Fig. 9A data for increased tumour regression in mice caused by expression of the fusion protein of the invention, and in
- Fig. 9B data for improved survival of tumour bearing mice caused by expression of the fusion protein of the invention.

In the figures, * denotes significance, ** higher significance, *** even more pronounced significance, n.s. denotes no significance. In the examples, the viral particles were based on the oncolytic adenovirus Ad5/11 carrying the deletion of ΔE1A and the p53-dependent expression of RNAi, essentially leading to expression of viral protein in p53-deficient tumour cells and limited levels of viral protein in p53-normal cells. This preferred viral particle is encoded by SEQ ID NO: 13 or consists thereof, wherein the coding sequence for the fusion protein of nucleotides 4405-5985 can be replaced by a nucleotide sequence encoding an embodiment of the fusion protein of the invention, e.g. encoding one of SEQ ID NO: 1, SEQ ID NO: 2, SEQ ID NO: 3 and SEQ ID NO: 4.

### Example 1: activating T-cells by presence of the fusion protein in vitro

As an example for a fusion protein without oligomerizing domain, a fusion protein consisting of secretory leader peptide - murine anti-CD3-scFv - hinge - HIV-Tat and, separately as an example for a fusion protein containing an oligomerizing domain a fusion protein consisting of secretory leader peptide - murine anti-CD3-scFv - Tag - hinge - oligomerizing domain - HIV-Tat were produced in HEK-293 cells. These fusion proteins had an amino acid sequence corresponding to SEQ ID NO: 9 as e.g. encoded by SEQ ID NO: 11, or SEQ ID NO: 10 as e.g. encoded by SEQ ID NO: 12, respectively, containing an anti-murine CD3 scFv instead of the anti- human CD3 scFv of SEQ ID NO: 1 and 3.

In short, HEK-293 cells were cultivated and transfected by using polyethyleneimine (PEI) with a nucleic acid construct comprising SEQ ID NO: 11 or SEQ ID NO: 12, encoding one of the fusion proteins and then cultivated for 72 h. The fusion proteins were isolated from the cell culture supernatant by Ni-affinity chromatography using a Ni-NTA column. The purified fusion proteins were added to separate aliquots of freshly isolated murine splenocytes and incubated under cell culture conditions for 24 h. In short, splenocytes were isolated from spleens of mice and one of the fusion proteins was added at amounts of 0.5µg, 1µg, 5µg or 10µg, respectively for 1mL splenocyte culture medium.

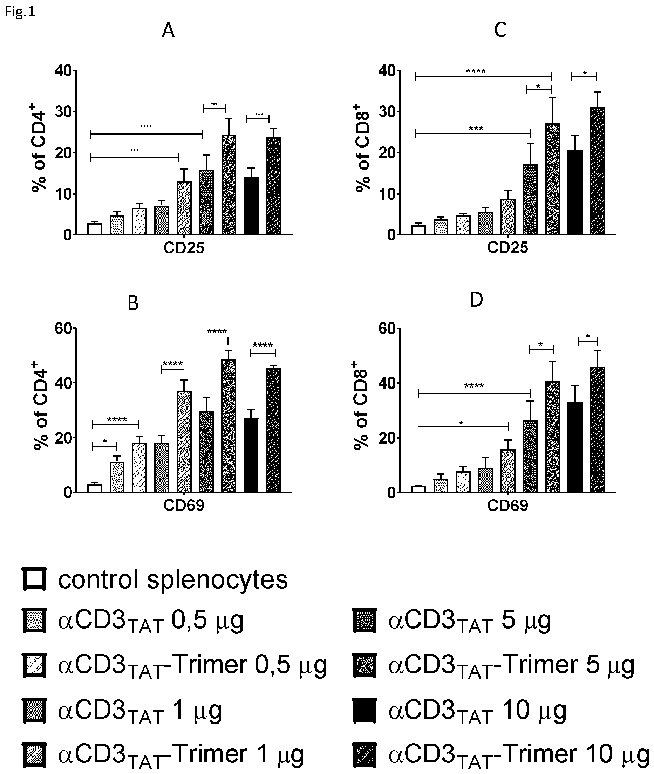

Subsequent to the incubation of splenocytes with the fusion protein, the splenocytes were analyzed by flow cytometry (FACS) for the activation markers CD25 and CD69 on CD4+ T-cells and on CD8+ T-cells for CD90.2+ leukocytes. The results are depicted in Fig. 1A and 1B for CD4+ T-cells and in Fig. 1C and 1D for CD8+ T-cells, for the fusion protein without oligomerizing domain (αCD3_{TAT}), and for the fusion protein with the fibritin oligomerizing domain (αCD3_{TAT}-Trimer). The results show that the percentage of both CD4+ and CD8+ T-cells having the activated CD25+ and CD69+ increased with increasing concentrations of added fusion protein. Further, it is shown that the trimerizing embodiment of the fusion protein, which contains the fibritin oligomerization domain, at the same protein concentration induced an increased activation of both CD4+ and CD8+ T-cells.

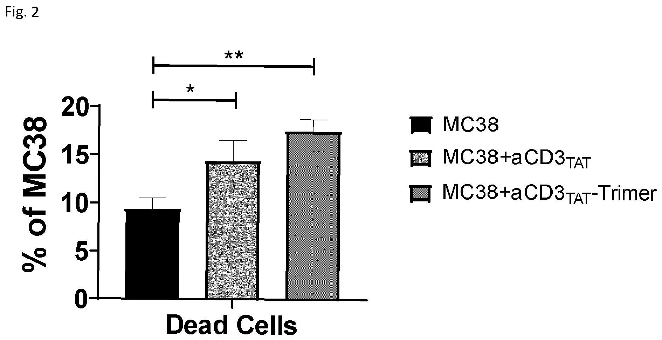

Further, 1 × 10⁶ freshly isolated murine splenocytes supplemented with one of the fusion proteins consisting of secretory leader peptide -anti- murine CD3-scFv - hinge - HIV-Tat or secretory leader peptide -anti- murine CD3-scFv - Tag - hinge - oligomerizing domain - HIV-Tat at a total amount of 1 µg in a total volume of 1 mL culture medium were added to 1 × 10⁵ murine colon cancer cells (MC38) resulting in an effector : target cell ratio of 10:1. After 24 h co-incubation under cell culture conditions, the number of surviving cancer cells was determined by flow cytometry. Fig. 2 shows that both fusion proteins resulted in an increased killing of cancer cells, and that the fusion protein containing the oligomerization domain resulted in increased killing of tumour cells. For the MC38 tumour cells, the fusion protein containing the fibritin oligomerization domain (Fig. 2: MC38 + αCD3_{TAT}-Trimer) was used at the same protein concentration as used for the embodiment without the oligomerizing domain (secretory leader peptide -anti- murine CD3-scFv - hinge - HIV-Tat, Fig. 2: MC38 + αCD3_{TAT}). Accordingly, this effective activation of T-cells showed a significantly higher molar activating effect for the embodiment containing an oligomerizing domain.

### Example 2: activation of human T-cells from PBMC by a fusion protein

Human T-cells were isolated from human whole blood as PBMC by pancoll gradient centrifugation as a buffy coat layer. The activation of the human T-cells was analysed by determining the CD69 as an activation marker by FACS on CD4+ and CD8+ T-cells after incubation for 24 h of PBMC in 1 mL cell culture medium with 1 µg fusion protein consisting of secretory leader peptide -anti- human CD3-scFv - hinge - HIV-Tat (SEQ ID NO: 1, αCD3h_{TAT}, or alternatively SEQ ID NO: 3) or secretory leader peptide -anti- human CD3-scFv - Tag - hinge - oligomerizing domain - HIV-Tat (SEQ ID NO: 2, αCD3h_{TAT}-Trimer, or alternatively SEQ ID NO: 4). During FACS, in addition to a labelled anti-CD69 antibody, labelled anti-CD4 antibody, anti-CD8 antibody, anti-CCR7 antibody and anti-CD45RA antibody were used, TNFα was determined by intracellular immunologic cytokine staining (ICS), and anti-c-myc antibody was used for specific staining of the fusion protein.

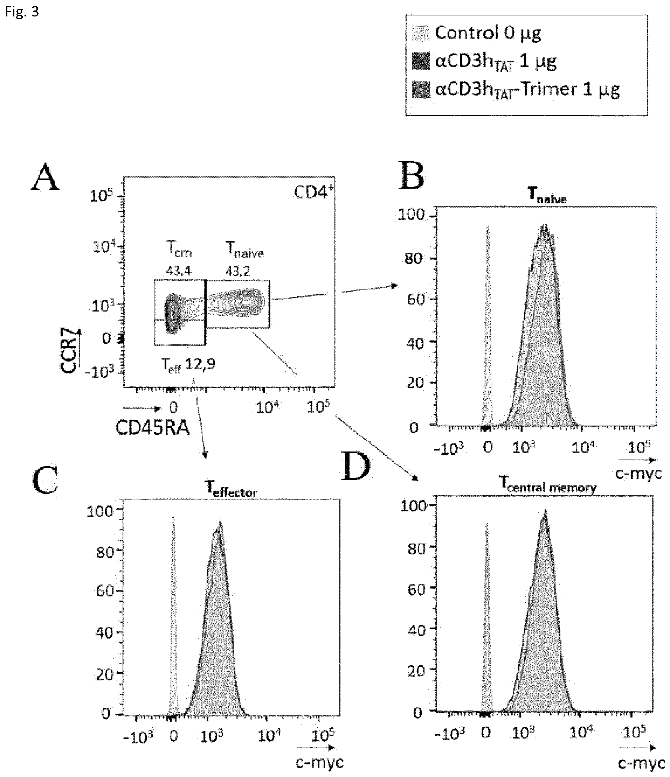

The FACS results depicted in Fig. 3A-D show that subtypes of human CD4+ T-cells, specifically naive T-cells, T effector, and central memory T-cells were associated with the fusion protein as shown by binding of anti-c-myc antibody.

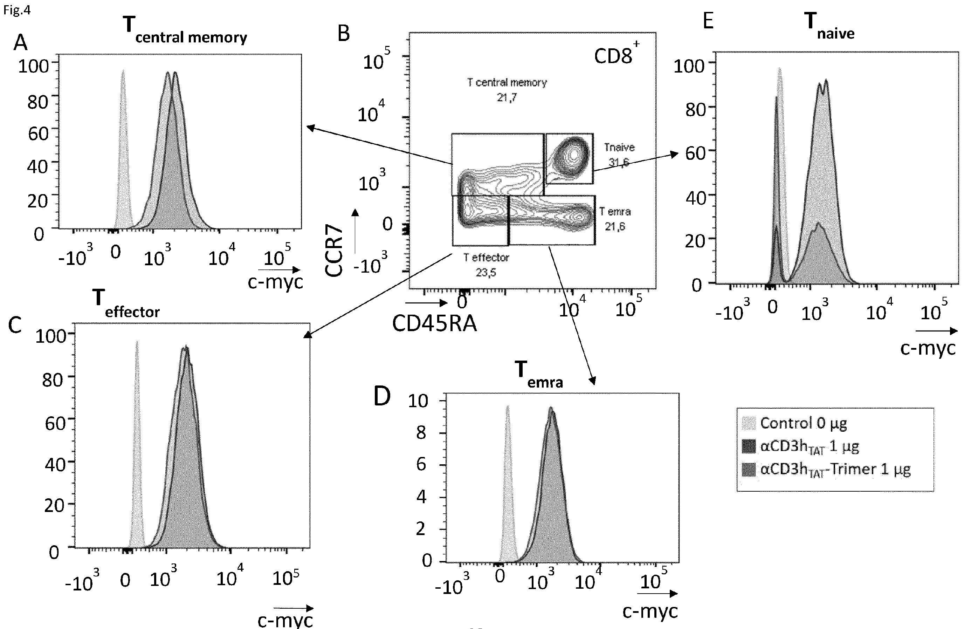

The FACS results depicted in Fig. 4A-E show that also subtypes of human CD8+ T-cells, specifically naive T-cells, T effector, T EMRA, and central memory T-cells were associated with the fusion protein as shown by binding of anti-c-myc antibody.

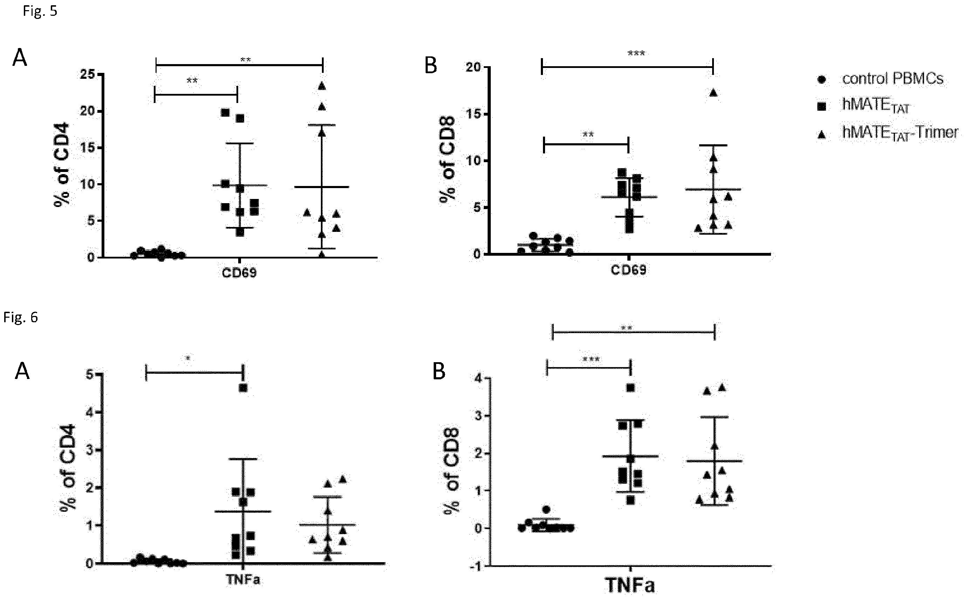

The FACS results depicted in Fig. 5A-B for anti-CD69 antibody staining show that both CD4+ and CD8+ T-cells were activated by each embodiment of the fusion proteins, consisting of secretory leader peptide - anti-human-CD3-scFv - hinge - HIV-Tat (SEQ ID NO: 1, hMATE_{TAT}) or secretory leader peptide - murine anti-CD3-scFv - hinge - HIV-Tat or secretory leader peptide -anti-human-CD3-scFv - Tag - hinge - oligomerizing domain - HIV-Tat (SEQ ID NO: 2, hMATE_{TAT}-Trimer).

The FACS results depicted in Fig. 6A-B for intracellular cytokine staining for TNFα (TNFa) shows cytokine production by CD4+ and CD8+ T-cells activated by one of the fusion proteins.

In summary, these results show that the fusion protein of the invention effectively binds to CD4+ and to CD8+ T-cells, including several subtypes of the T-cells, and that binding of the fusion protein strongly activates both CD4+ and CD8+ T-cells, including activating production of cytotoxic cytokines.

### Example 3: Treatment of a solid tumour by oncolytic adenovirus expressing the fusion protein

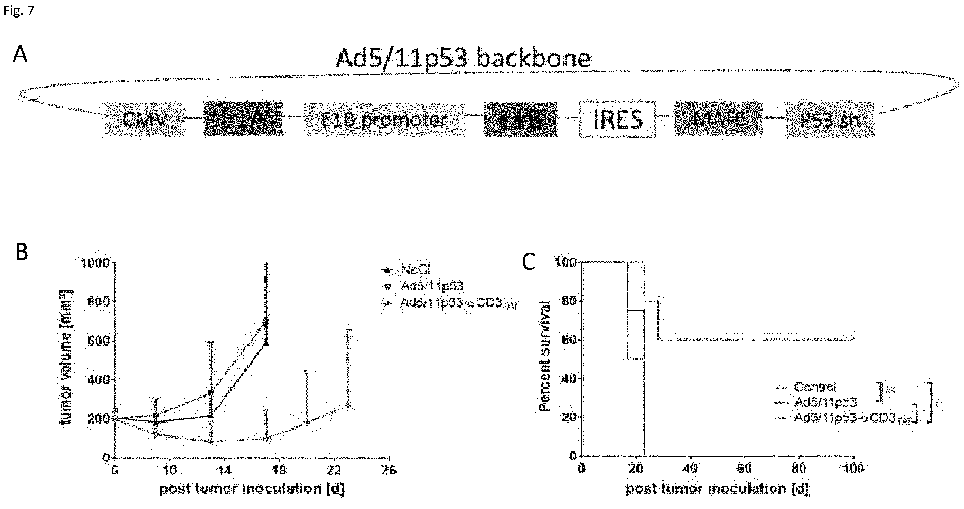

As a generally preferred example for an oncolytic virus, also referred to as oncolytic viral particle, an adenovirus serotype 5 (Ad5/11-p53) was used, which in dependence on presence of p53 in normal, non-cancer cells expresses shRNA directed against binding sites in its nucleic acid construct that are arranged to avoid expression of viral genes in normal cells, while allowing expression of encoded genes and replication in tumour cells that have lower expression of p53. Further, this oncolytic virus carried an inactivated E4 orf3 locus and a mutated E4 orf4. A preferred embodiment of this oncolytic virus is encoded by SEQ ID NO: 13, which encodes a fusion protein of the invention in the embodiment without oligomerization domain. This oncolytic virus was found to be specific for tumour cells, especially for use in the treatment of a tumour. A preferred oncolytic viral particle is adenovirus Ad5/11, which contains a nucleic acid sequence encoding the fusion protein, in this case encoding the fusion protein containing an anti-murine CD3 scFv in order to bind to the T cells of the experimental mouse. In this example, the fusion protein consisted of secretory leader peptide - anti-murine-CD3-scFv - hinge - HIV-Tat as an exemplary embodiment. A schematic of the viral particle encoding nucleic acid sequence is depicted in Fig. 7A, showing that the fusion protein encoding sequence is arranged under the translational control of an IRES and transcriptionally controlled by the E1B promotor. As generally preferred, this viral particle in dependence on presence of p53 generates shRNA that inhibit replication of the viral particle itself, e.g. in healthy tissue that expresses p53.

As an example for treatment of a solid tumour, immune-competent C57BL/6 mice were subcutaneously injected into the right flank with 1×10⁷ syngeneic colon adenocarcinoma cells (MC38), and mice were kept for 6 d, e.g. until tumour growth of 200 mm³ was detected.

For comparison to viral particles (Ad5/11p53-αCD3_{TAT}) according to SEQ ID NO: 13, saline (NaCl) as a negative control, or viral particles without the fusion protein (Ad5/1 1p53) were used, corresponding to SEQ ID NO: 13 but without the coding sequence for the fusion protein (i.e. without nucleotides No. 4405-5985 of SEQ ID NO: 13). The adenovirus containing the DNA of SEQ ID NO: 13 and the comparative adenovirus were separately produced, e.g. in cultivated HEK293 cells. These viral particles were isolated from the culture supernatant and intratumorally injected at a single dose of 1×10⁹.

Fig. 7B shows the tumour volume in mice, demonstrating that the comparative adenovirus (Ad5/11p53) resulted in decreased tumour growth compared to saline (NaCl), and that the viral particle encoding a fusion protein of the invention (Ad5/11p53-αCD3_{TAT}) resulted in drastically lower tumour growth, also in comparison to the comparative adenovirus.

Fig. 7C shows the survival of tumour-bearing mice after treatment with saline (Control), with the comparative adenovirus (Ad5/11p53) that does not encode the fusion protein, and treatment with an otherwise identical viral particle (SEQ ID NO: 13, Ad5/11p53-αCD3_{TAT}) encoding the fusion protein.

The comparison of the anti-tumour effect of the viral particle encoding the fusion protein to the comparative adenovirus shows that the fusion protein significantly increases the anti-tumour activity of the viral particle.

### Example 4: Treatment of a solid tumour by oncolytic adenovirus expressing the fusion protein under the control of a weak promoter

As a generally preferred example for an oncolytic virus, also referred to as oncolytic viral particle, an adenovirus serotype 5, encoding a fusion protein of the invention, represented by SEQ ID NO: 13 Ad5/11p53-αCD3_{TAT} was used for testing the influence of the promoter strength driving transcription of the fusion protein encoding nucleic acid sequence. As a representative of a strong promoter, the CMV promoter was arranged to control transcription of the fusion protein encoding DNA section, replacing the IRES in SEQ ID NO: 13 arranged in 5' to the fusion protein encoding DNA section. An expression unit comprising an IRES-motif arranged in 5' of the fusion protein encoding nucleic acid section was used to achieve a weak expression similar to the use of a weak promoter controlling transcription of the fusion protein encoding DNA section as shown in SEQ ID NO: 13. As a further example, the fusion protein encoding DNA section in SEQ ID NO: 13 was replaced by a section encoding a fusion protein containing an oligomerization domain according to amino acid SEQ ID NO: 2 or SEQ ID NO: 4, in each embodiment under the control of the IRES.

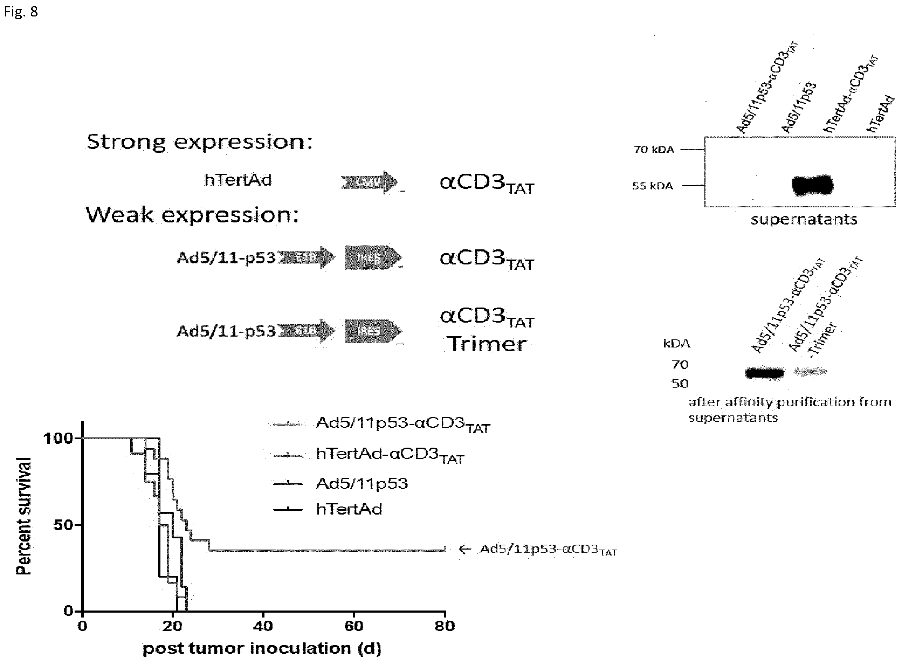

Fig. 8A schematically depicts the nucleic acid constructs, for strong expression containing the CMV promoter (CMV, hTertAd) or for weak expression containing the IRES (Ad5/11-p53), controlling transcription of the fusion protein without oligomerization domain, the fusion protein consisting of secretory leader - anti-human CD3 scFv - MycTag - HisTag - hinge - HIV-Tat (αCD3_{TAT}, e.g. SEQ ID NO: 1 or SEQ ID NO: 3), or under the control of the weak promoter controlling transcription of the fusion protein containing an oligomerization domain, the fusion protein consisting of secretory leader - anti-human CD3 scFv - MycTag - HisTag - hinge - fibritin domain - hydrophilic spacer - HIV-Tat (αCD3_{TAT}Trimer, e.g. SEQ ID NO: 2 or SEQ ID NO: 4), wherein the fibritin domain represents an oligomerization domain and wherein the hydrophilic spacer is optional. A preferred embodiment of this oncolytic virus is encoded by SEQ ID NO: 71, which encodes a fusion protein of the invention in the embodiment containing a fibritin domain as an oligomerization domain.

Fig. 8B, upper picture, shows a Western blot of culture supernatants of HEK 293 cells at 48 h after infection by the viral particles encoding a fusion protein of the invention, with immune detection using a labelled anti-Myc antibody. For a sample of 50 µL protein of each supernatant, the Western blot shows in lane 1 the viral particle encoding the fusion protein under the control of the E1B promoter and an IRES-motif for expression of the fusion protein (Ad5/11p53-αCD3_{TAT}) of the invention, lane 2 as control a similar viral particle without encoding a fusion protein of the invention (Ad5/11p53), lane 3 the viral particle encoding the fusion protein under direct control of a strong promoter, represented by the CMV promoter (hTertAd-αCD3_{TAT}), lane 4 as a control the similar viral particle containing a strong promoter (CMV promoter) without encoding a fusion protein of the invention (hTertAd). This shows that the viral particle encoding the fusion protein under the control of a strong promoter yields high expression of the fusion protein, whereas in this experiment, expression of the fusion protein under the control of the E1B promoter and an IRES motiv was not sufficient for direct detection in the supernatant indicating a low expression level.

Fig. 8B, lower picture, shows a Western blot of protein samples obtained from cell culture supernatants after protein purification by metal ion affinity chromatography for targeted enrichment of fusion proteins. Supernatants were obtained from 293 T-cells at 48 h after infection by the viral particles encoding a fusion protein of the invention, and immune detection was performed using a labelled anti-Myc antibody. For a sample of 50 µL of the purified protein, lane 1 (unlabeled) shows the purified protein the of an empty virus (Ad5/11p53) lacking expression of a fusion protein, lane 2 shows protein purified after infection with the viral particle encoding the fusion protein under the control of the E1B promoter/IRES (Ad5/11p53-αCD3_{TAT}) of the invention, lane 3 for the viral particle encoding the fusion protein containing an oligomerization domain under the control of the E1B promoter/IRES (Ad5/11p53-αCD3_{TAT}-Trimer) of the invention.

This positive detection after concentrating the fusion protein of the invention proves that the fusion protein of the invention is present in soluble form in supernatants of infected cells when expressed under the control of a weak expression unit (E1B promoter linked with an IRES), both for the embodiment without an oligomerization domain, and for the embodiment containing an oligomerization domain.

Fig. 8C shows the survival rate of C57BL/6 mice bearing the MC38 tumour as described in Example 3, with intratumoral injection of these viral particles. The result shows that the expression of a fusion protein of the invention under the control of a weak promoter results in a drastically improved survival, i.e. tumour treatment, e.g. in comparison to treatment with an oncolytic viral particle encoding the same fusion protein under the control of a strong promoter and in comparison to the oncolytic viral particle without the fusion protein (SEQ ID NO: 13 lacking the nucleic acid sequence encoding the fusion protein).

### Example 5: Treatment of a solid tumour by oncolytic adenovirus expressing the fusion protein

As a generally preferred example for an oncolytic virus, also referred to as oncolytic viral particle, an adenovirus serotype 5, encoding a fusion protein of the invention, represented by SEQ ID NO: 13 Ad5/11p53 was used for testing, encoding a fusion protein of the invention without oligomerization domain (αCD3_{TAT}) and in an embodiment of the fusion protein containing an oligomerization domain (αCD3_{TAT}-Trimer). C57BL/6 mice bearing the MC38 tumour were used as described in Example 3.

The viral particles were based on the oncolytic adenovirus Ad5/11 carrying the deletion of ΔE1A and the p53-dependent expression of RNAi according to SEQ ID NO: 13, encoding a fusion protein without oligomerization domain e.g. according to SEQ ID NO: 9 (Ad5/11p53AE1A- αCD3_{TAT}), or encoding a fusion protein that includes an oligomerization domain e.g. according to SEQ ID NO: 10 (Ad5/11p53ΔE1A- αCD3_{TAT}-Trimer). As a negative control, 0,9 % saline (NaCl) and the viral particle without coding sequence for a fusion protein (Ad5/11p53ΔE1A) were used.

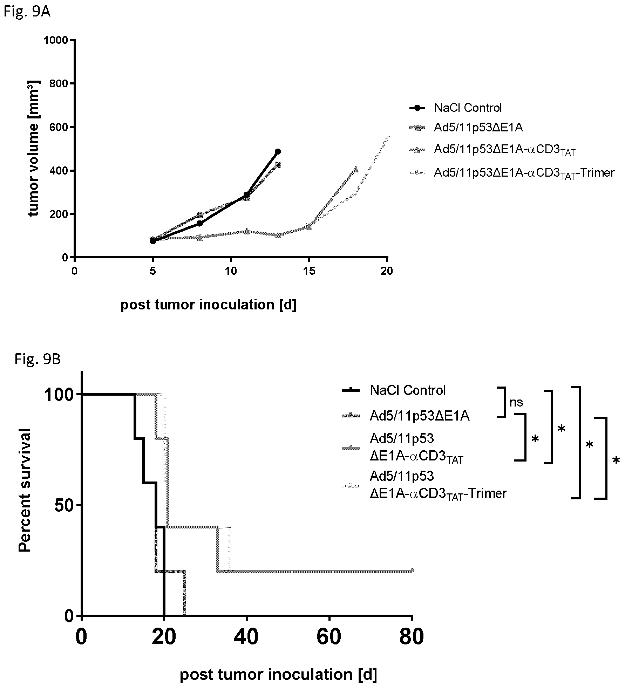

Fig. 9A shows that the tumour volume was significantly lower for viral particles expressing one of the fusion proteins of the invention, and Fig. 9B shows that survival, i.e. tumour treatment were significantly improved for viral particles expressing one of the fusion proteins of the invention, with a better effect for the fusion protein containing the oligomerization domain.

## Claims

1. Fusion protein comprising an anti-CD3 antibody section, **characterized in that** the fusion protein from N-terminus to C-terminus comprises or consists of an anti-CD3 antibody section, a hinge and a HIV-Tat domain.

2. Fusion protein according to claim 1, **characterized in that** the fusion protein comprises an oligomerizing domain, preferably in combination with a hydrophilic spacer, arranged between the hinge and the HIV-Tat domain.

3. Fusion protein according to claim 1, **characterized in that** the oligomerizing domain is a fibritin domain or a GCN4 domain.

4. Fusion protein according to claim 3, **characterized in that** the fibritin domain has an amino acid sequence of at least 80% homology or identity to at least one of amino acids 521..550 of SEQ ID NO: 2, and amino acids 521..550 of SEQ ID NO: 4.

5. Fusion protein according to one of the preceding claims, **characterized in that** the hinge has an amino acid sequence of at least 80% homology or identity to at least one of amino acids 285..520 of SEQ ID NO: 1, amino acids 285..520 of SEQ ID NO: 2, amino acids 285..520 of SEQ ID NO: 3, and amino acids 285..520 of SEQ ID NO: 4.

6. Fusion protein according to one of the preceding claims, **characterized in that** the HIV-Tat domain has least 80% homology or identity to at least one of amino acids 521..530 of SEQ ID NO: 1, amino acids 557..566 of SEQ ID NO: 2, amino acids 521..530 of SEQ ID NO: 3, and amino acids 557..566 of SEQ ID NO: 4.

7. Fusion protein according to one of the preceding claims, **characterized in that** the anti-CD3 antibody section is an anti-CD3 scFv specific for human CD3.

8. Fusion protein according to one of the preceding claims for use in tumour treatment, especially for use in treatment of a solid tumour.

9. Nucleic acid construct encoding an anti-CD3 antibody section for use in tumour treatment, **characterized in that** the nucleic acid construct encodes a fusion protein according to one of the preceding claims.

10. Nucleic acid construct according to claim 9, **characterized in that** the nucleic acid construct is contained in a viral particle.

11. Nucleic acid construct according to claim 9, **characterized in that** the nucleic acid construct is contained in a liposome.

12. Nucleic acid construct according to one of claims 9 to 11, **characterized in that** the nucleic acid construct is contained in an adenoviral particle.

13. Nucleic acid construct according to one of claims 9 to 12, **characterized in that** the nucleic acid construct comprises a nucleotide section of nucleotides 1..3706 and a nucleotide section of nucleotides 5986..37135 of SEQ ID NO: 13 and between these nucleotide sections comprises a coding sequence encoding the fusion protein under the control of a promoter, or comprises a nucleotide section of nucleotides 1..3706 and a nucleotide section of nucleotides 6094..37243 of SEQ ID NO: 71 and between these nucleotide sections comprises a coding sequence encoding the fusion protein under the control of a promoter.

14. Nucleic acid construct according to one of claims 9 to 13, **characterized in that** the coding sequence encoding the fusion protein is arranged under the control of a weak promoter.

15. Nucleic acid construct according to one of claims 9 to 13, **characterized in that** the coding sequence encoding the fusion protein is arranged in 3' of an IRES, with a promoter arranged in 5' of the IRES.
